# FASCICULE DE BREVET EUROPEEN

(11) **EP 3 710 799 B1**
(45) Date de publication et mention de la délivrance du brevet: **05.01.2022**
(21) Numéro de dépôt: 18819325.4
(22) Date de dépôt: 15.11.2018
(51) Int. Cl.: A61N 5/06, A61B 90/00, G01L 1/20, A61B 5/00, A61M 35/00, A61B 5/0531, A45D 34/04

(54) **APPLICATEUR DE PRODUIT FLUIDE**
APPLIKATOR FÜR FLÜSSIGE PRODUKTE
FLUID PRODUCT APPLICATOR

(30) Priorité: 16.11.2017 FR 1760812
(43) Date de publication de la demande: 23.09.2020
(73) Titulaire: Aptar France SAS, 27110 Le Neubourg (FR)
(72) Inventeur: CHEN, Alexandre, 77124 Villenoy (FR)
(74) Mandataire: CAPRI
(86) Numéro de dépôt international: PCT/FR2018/052859
(87) Numéro de publication internationale: WO 2019/097173

(56) Documents cités:
- US-A1- 2010 090 299
- US-A1- 2014 296 772
- US-A1- 2015 059 486

## Description

La présente invention concerne un applicateur de produit fluide comprenant un dispositif de détection de contact, et notamment un contact direct ou indirect avec la peau. Un domaine d'application privilégié de l'invention sera donc ceux de la cosmétique et de la pharmacie, et plus particulièrement celui des applicateurs de produit fluide, tel que la crème, la pommade, les gels, etc. Toutefois, d'autres domaines d'application où un contact étendu est mis en œuvre ne sont pas exclus.

Dans l'art antérieur, on connait déjà le document WO2013/121145 qui décrit un distributeur de produit fluide comprenant un réservoir de produit fluide, un organe de distribution de produit fluide connecté au réservoir, un organe d'actionnement pour actionner l'organe de distribution, un orifice de distribution de produit fluide connecté à l'organe de distribution, et une surface d'application pour appliquer le produit fluide issu de l'orifice de distribution sur la peau. Le distributeur comprend en outre au moins une source de rayonnement, telle qu'une diode électroluminescente LED, ayant une action anti-inflammatoire et/ou de stimulation des métabolismes de régénération de la peau. Plus généralement, ce document décrit un distributeur/applicateur de produit fluide intégrant des moyens d'activation du produit fluide et/ou de la peau.

Avec un tel distributeur/applicateur de produit fluide, il peut être intéressant de savoir à quel moment la surface d'application enduite de produit fluide est effectivement et/ou correctement en contact de la peau, afin de pouvoir activer la source de rayonnement. Il faut en effet éviter que le rayonnement de la source soit dirigé vers des organes sensibles, comme les yeux ou les muqueuses. Pour cela, différentes techniques de détection ont déjà été expérimentées, comme par exemple la détection IR, des capteurs d'ambiance, des contacteurs mécaniques, des micro-courants, etc. Toutefois, les résultats n'ont pas été suffisamment efficaces et fiables, notamment en raison de la présence du produit fluide entre la surface d'application de l'applicateur et la peau, qui perturbe la détection.

D'autre part, les écrans résistifs des smartphones auraient pu convenir en tant que détecteur de contact, sauf qu'il n'est pas possible de descendre en dessous de 1.7 pouce en diagonale et de détecter plusieurs zones d'appui en même temps.

Un des buts de la présente invention est donc de remédier à l'inconvénient précité de l'art antérieur en développant un nouveau dispositif de détection qui n'est pas sensible à la présence du produit fluide. Un autre du but de l'invention est de détecter un contact sur une zone ou aire étendue, afin d'éviter la détection de contact localisé ou ponctuel. Encore un autre but est de développer un dispositif de détection qui est utilisable avec des sources de rayonnement sans les perturber ou les atténuer significativement. Un autre but est de détecter le contact avec la peau (avec ou sans crème) sur un support transparent. Encore un autre but est développer un dispositif de détection qui est étanche au produit fluide.

Pour atteindre ces buts, la présente invention propose applicateur de produit fluide comprenant un dispositif de détection de contact, et notamment un contact avec la peau, comprenant un substrat externe souple définissant une surface externe de contact, le substrat externe souple recouvrant une zone de détection qui comprend plusieurs pastilles conductrices séparées les unes des autres, ces pastilles conductrices étant disposées au-dessus d'une dalle conductrice commune, les pastilles conductrices étant espacées, en l'absence de contrainte, de la dalle conductrice commune par des entretoises isolantes, de sorte qu'une pression exercée sur le substrat externe souple amène au moins une des pastilles conductrices à venir en contact avec la dalle conductrice commune, créant ainsi au moins un signal de court-circuit.

Chaque pastille conductrice peut ainsi créer un contact, et donc un court-circuit, avec la dalle commune sous-jacente. Le but n'est pas de localiser l'endroit de la pastille où le contact est créé, comme c'est le cas dans un écran tactile de Smartphone ou ordinateur, mais simplement de constater qu'un court-circuit a été généré au niveau d'une pastille déterminée. Ce constat est purement binaire : court-circuit ou non au niveau d'une pastille.

En identifiant ou discriminant chaque pastille de manière unique, il est ainsi possible d'identifier avec précision quelle est la ou les pastilles qui sont en contact avec la dalle commune.

D'autre part, en fonction du nombre de pastilles où un court-circuit est constaté à un moment précis, on peut en déduire que le contact est plus ou moins étendu.

On peut alors dire que le dispositif de détection de contact de l'invention permet non seulement de détecter un contact quelconque dans sa zone de détection, mais également de déterminer quelle est l'étendue ou l'ampleur du contact en terme de superficie ou d'aire. Cela donne une information supplémentaire ou complémentaire que l'on peut utiliser pour générer des actions ultérieures.

De même, le fait de découper ou segmenter la zone de détection en plusieurs sous-zones définies par les pastilles conductrices permet également de pouvoir cartographier avec précision la localisation du contact étendu en fonction du nombre et de l'emplacement des pastilles mises en court-circuit. On peut ainsi tracer un motif du contact étendu sur la zone de détection.

Il est aussi possible de mesurer la durée de chaque court-circuit et de corréler les durées de vie de l'ensemble des pastilles pour en déduire quelles sont les pastilles les plus court-circuitées, ce qui donne une information quant à l'endroit où le contact est le plus fréquent.

Il va de soi que le dispositif de détection de contact de l'invention peut être mis en œuvre dans n'importe quelle application nécessitant une détection de contact, allant de la simple présence d'un contact jusqu'à une analyse poussée de l'identification du contact, la localisation du contact, la durée du contact et l'étendue du contact.

Selon une caractéristique avantageuse, l'un au moins parmi les pastilles conductrices et les entretoises isolantes est souple. En d'autres termes, les pastilles peuvent être souples ou les entretoises peuvent être souples ou les deux à la fois. En pratique, les pastilles sont souples, puisque très fines et montées sur le substrat externe souple, et les entretoises sont dures, puisque constituées de résine et appliquée sur la dalle commune, qui est avantageusement montée sur un substrat de base rigide. Ainsi, le substrat externe souple est déformé par le contact avec un objet tiers, comme la peau, cette déformation affectant les pastilles, dont certaines ou toutes vont venir en contact avec la dalle commune rigide, créant ainsi un ou plusieurs courts-circuits.

Selon un aspect intéressant de l'invention, le dispositif de détection peut être associé à un logiciel de traitement qui délivre un signal de contact étendu, lorsqu'il reçoit un nombre seuil de signaux de court-circuit, ce nombre seuil étant au moins égal à 1 et au plus égal au nombre de pastilles conductrices. Lorsque ce nombre seuil est 1, on peut par exemple choisir la pastille centrale. On pourra par exemple choisir un nombre seuil de 2 pastilles. Les 2 pastilles peuvent être adjacentes ou peu éloignées pour détecter un contact peu étendu ou au contraire, elles peuvent être éloignées pour détecter un contact plus étendu.

Avantageusement, la surface externe de contact présente un profil autre que plan, notamment courbe. Ceci est possible, étant donné que le substrat externe est souple et qu'il n'est pas nécessaire de détecter le positionnement précis du contact générant le court-circuit.

D'autre part, le dispositif de détection est transparent aux rayonnements électromagnétiques et à la chaleur. Ainsi, il peut être utilisé dans des distributeurs/applicateurs générant des rayonnements électromagnétiques et/ou à la chaleur, le dispositif de détection étant traversé par le rayonnement et/ou la chaleur sans créer de pertes ou de perturbations significatives.

Selon une autre caractéristique de l'invention, la zone de détection présente une surface totale de l'ordre de 300 à 400 mm², avec des pastilles conductrices de moins de 100 mm². En comparaison, un écran tactile classique mesure plus de 600 mm².

La présente invention définit un applicateur de produit fluide comprenant un dispositif de détection tel que défini ci-dessus. Selon une forme de réalisation, la surface externe de contact forme une surface d'application de produit fluide pour appliquer du produit fluide sur une surface cible, telle que la peau. En variante, l'applicateur comprend une paroi d'application de produit fluide qui est disposée en contact de la surface externe de contact.

Avantageusement, l'applicateur comprend une source d'activation du produit fluide et/ou de la peau et un logiciel de traitement intégré ou exporté, qui reçoit les signaux de court-circuit et les traitent pour obtenir au moins une des informations suivantes :
- identification des pastilles conductrices mises en court-circuit,
- repérage des pastilles conductrices mises en court-circuit dans la zone de détection,
- nombre de pastilles conductrices mises simultanément en court-circuit,
- durée du court-circuit de chaque pastille conductrice mise en court-circuit,
- rupture du court-circuit de chaque pastille conductrice mise en court-circuit,
afin d'en déduire au moins une des actions suivantes :
- déclenchement la source d'activation, dès lors qu'un nombre seuil de signaux de court-circuit simultanés a été détecté, avantageusement pendant une durée de temps fixée,
- coupure de la source d'activation, dès lors qu'un nombre seuil de signaux de court-circuit simultanés n'est plus détecté,
- affichage sur un écran, tel que de Smartphone, des informations relatives à l'identification, au repérage, au nombre et/ou à la durée des courts-circuits, par exemple sous la forme d'une représentation virtuelle de la zone de détection avec ses pastilles conductrices.

On voit ainsi que le dispositif de détection de l'invention trouve une application privilégiée dans un applicateur de produit fluide, car il permet non seulement de contrôler la source d'activation, mais également d'informer l'utilisateur sur la manière dont il utilise l'applicateur. L'utilisateur peut être averti qu'il utilise mal l'applicateur et il peut lui être proposé des solutions pour améliorer son utilisation. Le dispositif de détection de l'invention constitue donc une véritable source d'informations diverses et étendues qui peuvent être traitées au moyen de logiciels appropriés pour délivrer à l'applicateur et/ou à l'utilisateur des données affinées.

L'invention peut également s'appliquer à un distributeur de produit fluide comprenant un capot dont la présence/absence est repérée par le dispositif de détection de l'invention. Elle peut aussi s'appliquer à un distributeur de produit fluide comprenant un réservoir écrasable, la pression exercée par la main d'un utilisateur sur le réservoir écrasable étant perçue par le dispositif de détection de l'invention. On voit bien que le dispositif de détection de l'invention peut trouver des applications diverses et variées.

L'invention sera maintenant décrite plus en détail en référence aux dessins joints, donnant à titre d'exemple non limitatif, un mode de réalisation de l'invention et sa mise en œuvre dans un distributeur/applicateur de produit fluide.

Sur les figures :
La figure 1 est une vue schématique en perspective explosée du dispositif de détection de contact de l'invention,
La figure 2 est une vue en section transversale verticale à travers le dispositif de détection de contact, à l'état de repos,
La figure 3 est une vue similaire à celle de la figure 2 pour le dispositif de détection de contact, à l'état contraint,
La figure 4 est une vue très schématique montrant une zone de détection de forme trapézoïdale, et
La figure 5 est une vue en section transversale verticale à travers un distributeur/applicateur de produit fluide intégrant le dispositif de détection de contact de l'invention.

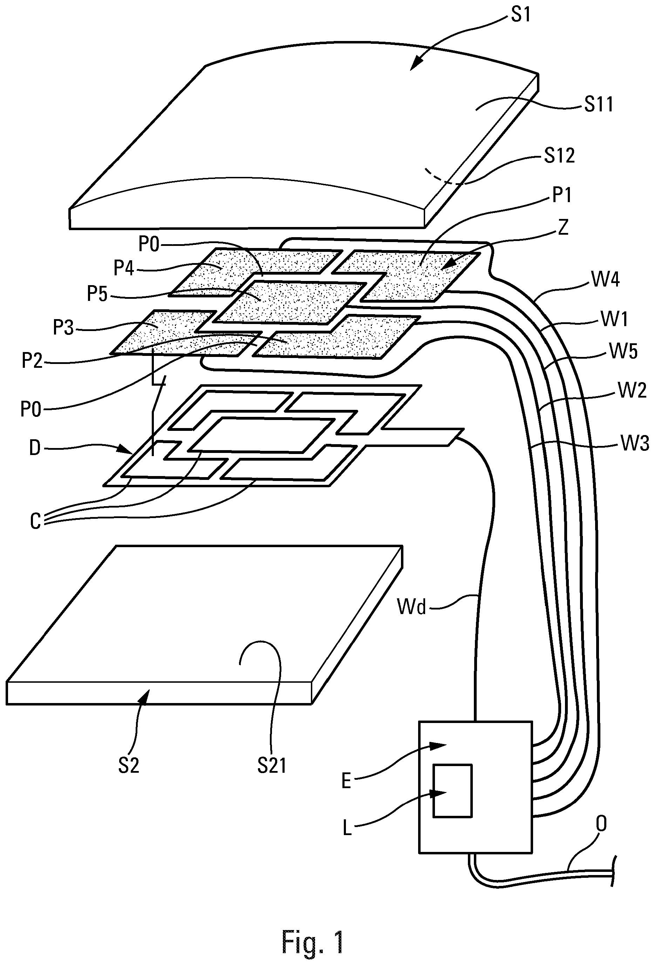

On se réfèrera tout d'abord aux figures 1 à 4 pour décrire la structure et le fonctionnement du dispositif de détection de contact de l'invention, selon un mode de réalisation non limitatif.

Le dispositif de détection de contact comprend un substrat de base S2, une dalle conductrice commune D, des entretoises isolantes C, plusieurs pastilles conductrices P1, P2, P3, P4 et P5 et un substrat externe S1.

Le substrat de base S2 n'est pas vraiment critique pour la présente invention et peut être constitué par n'importe quel support approprié. Dans certains cas, le substrat de base S2 peut même être considéré comme faisant partie d'un autre dispositif ou ensemble. Le substrat de base S2 est réalisé en un matériau isolant ou très faiblement conducteur, qui est de préférence rigide. Ce matériau peut être une matière plastique, par exemple un polymère thermoplastique transparent, tel que du Plexiglass ^{®}. Le substrat de base S2 définit une surface de support S21, qui peut être plane, comme visible sur la figure 1, ou non plane : elle peut par exemple être courbe ou bombée. L'épaisseur du substrat de base S2 peut être constante, comme visible sur la figure 1, ou au contraire variable. Le substrat externe S1 peut à la fois présenter une épaisseur constante et une forme courbe ou galbée. Il peut même présenter une forme complexe non géométrique.

Le substrat externe S1 peut être réalisé à partir de n'importe quel matériau isolant ou très faiblement conducteur, présentant une souplesse ou élasticité à mémoire de forme en rapport avec la force exercée par le contact à détecter. Son épaisseur est également déterminée en fonction de la force exercée par le contact à détecter. Le substrat externe S1 est de préférence transparent aux rayonnements électromagnétiques et/ou à la chaleur. Ce matériau peut être une matière plastique, par exemple du poly(téréphtalate d'éthylène) (PET). Le substrat externe S1 définit une surface externe de contact S11, qui peut être plane ou non plane : elle peut par exemple être courbe ou bombée, comme visible sur la figure 1. Le substrat externe S1 définit aussi une surface de montage S12, opposée à la surface externe de contact S11. La surface de montage S12 est de préférence plane. L'épaisseur du substrat externe S1 peut être constante ou au contraire variable, comme visible sur la figure 1. Le substrat externe S1 peut à la fois présenter une épaisseur constante et une forme courbe ou galbée. Il peut même présenter une forme complexe non géométrique.

La dalle conductrice commune D est monolithique, c'est-à-dire formée d'un seul tenant ou de plusieurs parties jointives. Elle est de préférence plane, comme la surface de support S21, mais elle peut également être courbe, bombée, concave ou de forme complexe non géométrique. La dalle conductrice commune D présente sur la figure 1 une forme générale carrée ou rectangulaire. D'autres formes géométriques ou non sont possibles. La dalle conductrice commune D est réalisée à partir d'un matériau électriquement conducteur. En pratique, elle peut être formée par dépôt d'une fine couche d'oxyde de métal, tel que d'indium-étain, sur la surface de support S21, avec une épaisseur de l'ordre de 5 à 100 microns, avantageusement de l'ordre de 5 à 10 microns et de préférence de 5 ± 1 microns. Plus l'épaisseur est fine et plus elle est transparente.

Les pastilles conductrices sont au nombre de 5 et désignées P1, P2, P3, P4 et P5 sur les figures. Bien entendu, ce nombre n'est pas limitatif et peut varier de 1 à l'infini. En pratique, on peut envisager de 2 à 100 pastilles, avantageusement de 4 à 20 pastilles. Elles sont de préférence planes, comme la surface de montage S12, mais elles peuvent également être courbes, bombées, concaves ou de forme complexe non géométrique. Les pastilles conductrices sont réalisées à partir d'un matériau électriquement conducteur. En pratique, elles peuvent être formées par dépôt d'une fine couche d'oxyde de métal, tel que d'indium-étain, sur la surface de montage S12, avec une épaisseur de l'ordre de 5 à 100 microns, avantageusement de l'ordre de 5 à 10 microns et de préférence de 5 ± 1 microns. Plus l'épaisseur est fine et plus elle est transparente.

On voit sur la figure 1 que les pastilles conductrices P1, P2, P3, P4 et P5 présentent une forme d'ensemble carrée ou rectangulaire, avec les pastilles séparées les unes des autres par plages étroites P0. L'ensemble de pastilles forme une zone de détection Z dont la superficie est inférieure ou égale à celle de la dalle conductrice commune D. Sur la figure, il y a quatre pastilles périphériques P1, P2, P3 et P4 en forme d'équerre ou de L et une pastille centrale P5 de forme carrée ou rectangulaire. Les plages P0 forment un cadre central de forme carrée ou rectangulaire avec quatre branches qui partent à partir du milieu de chaque côté du cadre central. La largeur des plages P0 est sensiblement constante.

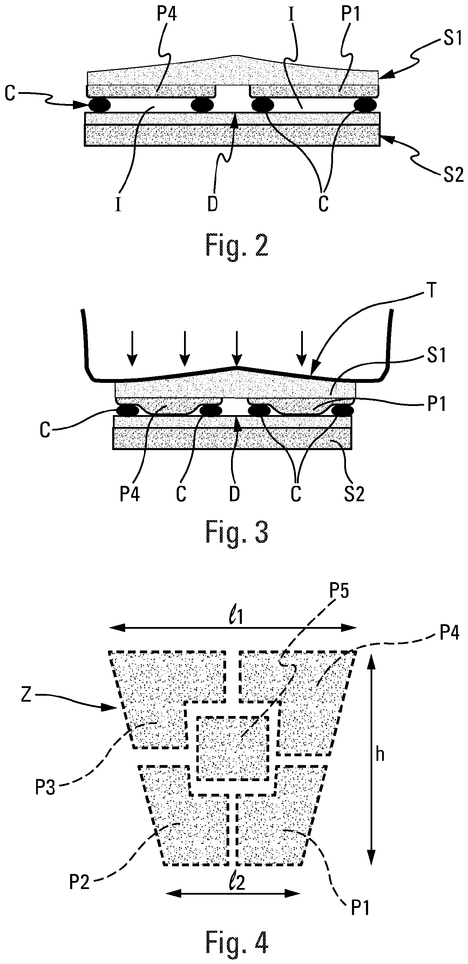

Sur la figure 2, on peut voir que les pastilles conductrices P1 et P4 sont espacées de la dalle conductrice commune D par des entretoises C, de manière à former entre elles des interstices I. Ces entretoises C sont réalisées à partir d'un matériau isolant, telle qu'une résine, qui peut être appliquée sur la dalle conductrice commune D, comme représenté sur la figure 1, où l'on peut voir cinq cadres de résine ayant des formes respectivement identiques à celles des pastilles conductrices P1 à P5. En variante, les entretoises C peuvent être déposées sur les pastilles conductrices. L'épaisseur des entretoises C est de l'ordre de 5 à 30 microns, avantageusement de l'ordre de 10 à 20 microns et de préférence de 15 ± 2 microns. Les interstices I présente une épaisseur en rapport avec l'épaisseur des entretoises C : ils peuvent être légèrement moins épais que les entretoises, par exemple de l'ordre de 1 à 3 microns, en raison de la déformabilité du substrat externe S1 et des pastilles P1 à P5.

La taille de la zone de détection Z peut être relativement petite, comparée aux écrans tactiles classique de Smartphones. Elle peut présenter moins de 20 mm de côté, correspondant à une superficie totale moins de 400 mm². La figure 4 représente une zone de détection Z qui est particulière, en ce sens qu'elle n'est pas de forme carrée ou rectangulaire, mais de forme trapézoïdale, avec un côté supérieur ayant une largeur Il de 20 mm, un côté inférieur ayant une largeur I2 de 15 mm, et une hauteur h de 20 mm. Avec ces dimensions, les pastilles présentent une superficie inférieure à 100 mm², et certaines même inférieure à 60 mm².

Enfin, des fils, câbles ou pistes conducteurs W1 à W5 relient respectivement chacune des pastilles P1 à P5 à une électronique de traitement et de contrôle E qui intègre avantageusement un logiciel de traitement L. Un fil, câble ou piste conducteur Wd de même type relie la dalle conductrice commune D à l'électronique de traitement et de contrôle E.

La figure 2 représente le dispositif de détection de contact au repos, c'est-à-dire qu'il n'est soumis à aucune contrainte extérieure. Les pastilles conductrices P1 et P4 sont espacées de la dalle D par les entretoises C qui créent des interstices I. Une tension électrique est appliquée entre les pastilles et la dalle : il ne se passe rien, étant donné qu'il n'y pas de contact.

Sur la figure 3, le substrat externe S1 est maintenant en contact d'un corps extérieur T, qui peut être la peau d'un utilisateur. Ce corps T exerce une pression telle sur le substrat externe S1 qu'il se déforme, et avec lui les pastilles conductrices P1 et P4 qui viennent alors en contact avec la dalle D, créant deux courts-circuits.

Ce qui se passe au niveau des pastilles P1 et P4 est également valable pour les autres pastilles P2, P3 et P5. Les courts-circuits créés génèrent chacun un signal de court-circuit qui est envoyé à l'électronique de traitement et de contrôle E.

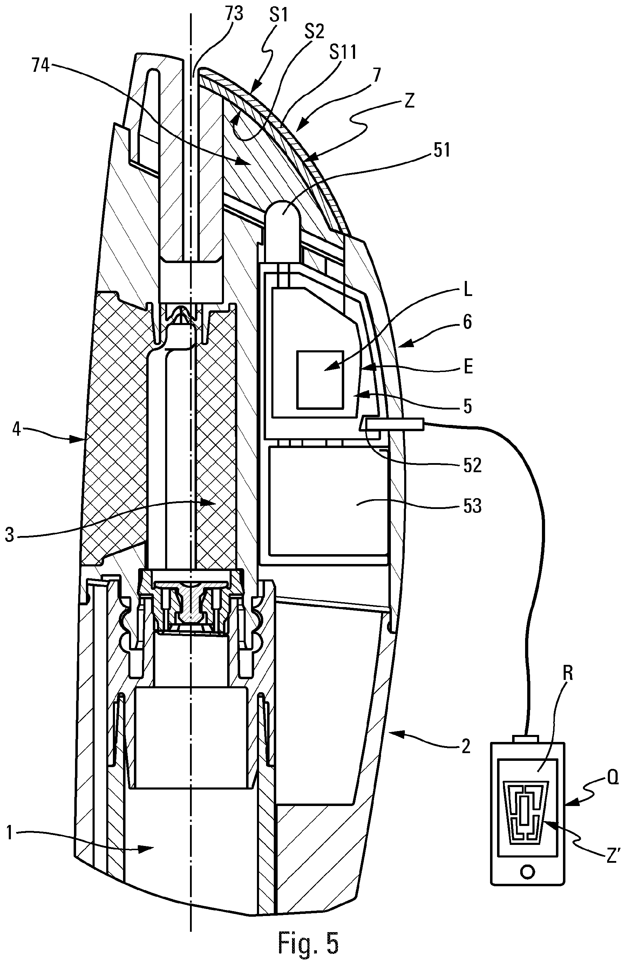

On se référera maintenant à la figure 5 pour voir de quelle manière le dispositif de détection de contact de l'invention peut être mis en œuvre dans un distributeur/applicateur de produit fluide. Le distributeur/applicateur comprend un réservoir de produit fluide 1, un étui 2 dans lequel le réservoir 1 est reçu, un organe de distribution 3 qui est ici une pompe, un organe d'actionnement 4 pour actionner la pompe 3 et qui est ici intégré à la pompe, un module 5 doté d'une (voire plusieurs) source de rayonnement 51, un corps 6 qui reçoit la pompe 3, l'organe d'actionnement 4 et le module 5, et enfin une tête de distribution 7 montée sur le corps 6. La tête 7 forme un orifice de distribution 73 et une surface d'application de produit fluide. Elle est également pourvue d'un guide d'onde 74 (par exemple une lentille) pour guider, amplifier ou focaliser la lumière émise par la source de rayonnement 51. Optionnellement, le distributeur peut encore comprendre un capot de protection qui coiffe la tête de distribution 7. Tous les éléments constitutifs du distributeur, à l'exception du module 5, peuvent être réalisés par injection moulage de matière plastique. On comprend qu'il est possible de distribuer du produit fluide à travers l'orifice de distribution 73 au niveau de la surface d'application en actionnant l'organe d'actionnement 4 de l'organe de distribution 3 qui permet de prélever du produit fluide du réservoir 1 et de le refouler à travers l'orifice de distribution 73. Une fois le produit distribué présent sur la surface d'application, l'utilisateur peut l'appliquer et l'étaler sur une surface cible, telle que la peau. Le produit fluide distribué est de préférence un produit visqueux, tels que de la crème, un gel, une pommade, etc.

Le module 5 peut se présenter sous la forme d'un petit boitier coiffé d'une ou de plusieurs LED(s), en tant que source de rayonnement 51. Le module comprend des moyens d'alimentation 53, par exemple sous la forme d'une batterie et une électronique de traitement et de contrôle E, par exemple montée sur une petite plaque de circuit imprimée. Cette électronique intègre avantageusement un logiciel de traitement L. Bien entendu, le module 5 peut intégrer encore d'autres composants électroniques aptes à remplir d'autres fonctions. Le module 5 comprend également un port 52, par exemple USB, pour le raccordement d'un Smartphone ou d'un ordinateur.

Sans sortir du cadre de l'invention, on peut prévoir, à la place des LED (s), d'autres sources d'activation qui vont agir sur le produit fluide et/ou sur la tissu (peau). On peut entres autres citer toutes les formes de rayonnement électromagnétiques (visibles ou non), les sources de chaleur, les vibrations ou les courants électriques (par exemple l'iontophorèse), etc.

Le dispositif de détection de contact de l'invention a été intégré dans cet applicateur au niveau de la tête de distribution 7. Plus en détail, la surface d'application, au moins dans sa partie située à droite de l'orifice de distribution 73 sur la figure 5, est formée par la surface externe de contact S11 du substrat externe S1 du dispositif de détection de contact de l'invention. En dessous du substrat S1 se trouve la zone de détection Z avec ses pastilles conductrices, puis la dalle commune D avec ses entretoises C montée sur le substrat de base S2. On peut constater sur la figure 5 que le dispositif de détection de contact dans son intégralité est courbe ou galbé avec des épaisseurs de parois constantes.

En variante non représentée, la surface externe de contact S11 pourrait être recouverte d'une paroi d'application de produit fluide formée par la tête 7. Cette paroi peut être souple. Elle peut aussi être rigide : elle peut alors être montée de manière flottante sur surface externe de contact S11.

Lorsqu'un utilisateur veux se servir de ce distributeur/applicateur, il commence par actionner la pompe 3 en appuyant sur le poussoir 4 avec l'orifice de distributeur 73 positionné à proximité de sa cible (peau), afin d'y déposer une dose de produit fluide. Ensuite, l'utilisateur va se servir de la surface externe de contact S11 pour étaler le produit fluide sur la cible. C'est à ce moment que le dispositif de détection de contact de l'invention entre en jeu. Les signaux de court-circuit générés par les contacts entre les pastilles et la dalle, du fait de la force de pression exercée au contact de la peau, sont acheminés au logiciel de traitement L qui les traitent pour obtenir diverses informations, dont les suivantes :
- identification des pastilles conductrices mises en court-circuit,
- repérage ou localisation des pastilles conductrices mises en court-circuit dans la zone de détection,
- nombre de pastilles conductrices mises simultanément en court-circuit,
- durée du court-circuit de chaque pastille conductrice mise en court-circuit, et
- rupture du court-circuit de chaque pastille conductrice mise en court-circuit.

Ces informations peuvent ensuite être corrélées afin d'en déduire au moins une des actions suivantes :
- déclenchement de l'alimentation de la source de rayonnement 51, dès lors qu'un nombre seuil de signaux de court-circuit simultanés a été détecté, avantageusement pendant une durée de temps fixée,
- coupure de l'alimentation de la source de rayonnement 51, dès lors qu'un nombre seuil de signaux de court-circuit simultanés n'est plus détecté,
- affichage sur un écran R, tel que de Smartphone Q, des informations relatives à l'identification, au repérage, au nombre et/ou à la durée des courts-circuits, par exemple sous la forme d'une représentation virtuelle Z' de la zone de détection Z avec ses pastilles conductrices P1 à P5.

Le déclenchement et la coupure de l'alimentation de la source de rayonnement 51 sont des actions internes qui influent directement sur le fonctionnement du distributeur/applicateur, dans le but d'assurer que la source de rayonnement 51 n'est déclenchée que lorsque la surface externe de contact S11 est en contact avec la peau de l'utilisateur et stoppée dès que la surface S11 est décollée de la peau. Cela permet d'éviter que la source de rayonnement 51 ne soit dirigée vers des tissus sensibles, tels que les yeux et/ou les muqueuses. Le nombre seuil de signaux de court-circuit est variable et peut aller de 1 jusqu'au nombre total de pastilles dans la zone de détection Z, sachant que ce nombre n'est pas limité en théorie.

Les autres actions sont externes, en ce sens qu'elles peuvent servir à l'utilisateur pour surveiller le déroulement de l'application du produit, soit en direct, soit en différé. L'utilisateur peut se servir des informations retraitées affichées sur l'écran R de son Smartphone Q pour corriger des défauts de manipulation du distributeur/applicateur et ainsi améliorer ses performances. Par exemple, si l'utilisateur voit que certaines pastilles sont presque toujours court-circuitées et d'autres presque jamais, il peut tenter de modifier l'orientation du distributeur/applicateur par rapport à la cible pour homogénéiser ou étendre le contact aux pastilles peu utilisées. L'écran R du Smartphone Q peut ainsi devenir l'organe de pilotage en direct du distributeur/applicateur.

Le logiciel de traitement L peut être entièrement embarqué dans le distributeur/applicateur, avantageusement dans son module 5. En variante, une partie du logiciel de traitement L peut être externalisé, par exemple sous la forme d'une application chargeable sur le Smartphone Q.

Le dispositif de détection de contact de l'invention peut également être mis en œuvre dans d'autres objets ou articles, par exemple pour détecter la présence d'un organe définissant une zone de contact étendue. La présence d'un capot ou d'un couvercle définissant une zone de contact annulaire peut être détectée, ce capot ou ce couvercle complétant par exemple un distributeur de produit fluide. La préhension par une main d'un réservoir écrasable d'un distributeur de produit fluide peut aussi être détectée à l'aide du dispositif de détection de contact de l'invention. L'invention trouvera bien entendu une application privilégiée partout où un contact étendu (en superficie) est recherché. Un contact ponctuel ou restreint peut toutefois être fiablement détecté avec l'invention.

Dans tous les cas, le dispositif de détection de contact de l'invention délivre des informations de base qui découlent directement du court-circuitage des diverses pastilles conductrices P1 à P5 : identification, nombre et localisation des pastilles court-circuitées, début, durée et fin du court-circuit. Ces informations de base sont recueillies par l'électronique de traitement et de contrôle E, et plus particulièrement par son logiciel L, qui les corrèle et les traite pour en tirer des informations complexes traitées qui vont servir à générer des actions internes, qui commandent le fonctionnement d'un appareil (distributeur ou autre) et des actions externes à l'usage de l'utilisateur, qui pourra s'en servir pour augmenter les performances de l'appareil pourvu du dispositif de détection de contact de l'invention.

## Revendications

1. Applicateur de produit fluide comprenant un dispositif de détection de contact, et notamment un contact direct ou indirect avec la peau, **caractérisé en ce que** le dispositif de détection de contact comprend un substrat externe souple (S1) définissant une surface externe de contact (S11), le substrat externe souple (S1) recouvrant une zone de détection (Z) qui comprend plusieurs pastilles conductrices (P1, P2, P3, P4, P5) séparées les unes des autres, ces pastilles conductrices (P1, P2, P3, P4, P5) étant disposées au-dessus d'une dalle conductrice commune (D), les pastilles conductrices (P1, P2, P3, P4, P5) étant espacées, en l'absence de contrainte, de la dalle conductrice commune (D) par des entretoises isolantes (C), de sorte qu'une pression exercée sur le substrat externe souple (S1) amène au moins une des pastilles conductrices (P1, P2, P3, P4, P5) à venir en contact avec la dalle conductrice commune (D), créant ainsi au moins un signal de court-circuit.

2. Applicateur selon la revendication 1, dans lequel l'un au moins parmi les pastilles conductrices (P1, P2, P3, P4, P5) et les entretoises isolantes (C) est souple.

3. Applicateur selon la revendication 1 ou 2, dans lequel la dalle conductrice commune (D) est montée sur un substrat de base rigide (S2).

4. Applicateur selon l'une quelconque des revendications précédentes, dans lequel la surface externe de contact (S11) présente un profil autre que plan, notamment courbe.

5. Applicateur selon l'une quelconque des revendications précédentes, étant transparent aux rayonnements électromagnétiques et à la chaleur.

6. Applicateur selon l'une quelconque des revendications précédentes, dans lequel la zone de détection (Z) présente une surface totale de l'ordre de 300 à 400 mm², avec des pastilles conductrices (P1, P2, P3, P4, P5) de moins de 100 mm².

7. Applicateur selon l'une quelconque des revendications précédentes, dans lequel la surface externe de contact (S11) forme une surface d'application de produit fluide pour appliquer du produit fluide sur une surface cible, telle que la peau.

8. Applicateur selon l'une quelconque des revendications précédentes, comprenant une paroi d'application de produit fluide qui est disposée en contact de la surface externe de contact.

9. Applicateur selon l'une quelconque des revendications précédentes, comprenant une source d'activation (51) du produit fluide et/ou de la peau et un logiciel de traitement (L) intégré ou exporté, qui est adapté à recevoir les signaux de court-circuit et à les traiter pour obtenir au moins une des informations suivantes :
- identification des pastilles conductrices (P1, P2, P3, P4, P5) mises en court-circuit,
- repérage des pastilles conductrices (P1, P2, P3, P4, P5) mises en court-circuit dans la zone de détection (Z),
- nombre de pastilles conductrices (P1, P2, P3, P4, P5) mises simultanément en court-circuit,
- durée du court-circuit de chaque pastille conductrice (P1, P2, P3, P4, P5) mise en court-circuit,
- rupture du court-circuit de chaque pastille conductrice (P1, P2, P3, P4, P5) mise en court-circuit,
afin d'en déduire au moins une des actions suivantes :
- déclenchement de la source d'activation (51), dès lors qu'un nombre seuil de signaux de court-circuit simultanés a été détecté, avantageusement pendant une durée de temps fixée,
- coupure de la source d'activation (51), dès lors qu'un nombre seuil de signaux de court-circuit simultanés n'est plus détecté,
- affichage sur un écran (R), tel que de Smartphone (Q), des informations relatives à l'identification, au repérage, au nombre et/ou à la durée des courts-circuits, par exemple sous la forme d'une représentation virtuelle (Z') de la zone de détection (Z) avec ses pastilles conductrices (P1, P2, P3, P4, P5).

10. Applicateur selon la revendication 9, dans lequel le logiciel de traitement (L) est adapté à délivrer un signal de contact étendu, lorsqu'il reçoit un nombre seuil de signaux de court-circuit, ce nombre seuil étant au moins égal à 2 et au plus égal au nombre de pastilles conductrices (P1, P2, P3, P4, P5).

## Patentansprüche

1. Applikator für flüssige Produkte, umfassend eine Vorrichtung zur Erkennung eines Kontakts, insbesondere eines direkten oder indirekten Kontakts mit der Haut, **dadurch gekennzeichnet, dass** die Vorrichtung zur Erkennung eines Kontakts ein flexibles äußeres Substrat (S1) umfasst, das eine äußere Kontaktfläche (S11) definiert, wobei sich das flexible äußere Substrat (S1) über einen Erfassungsbereich (Z) erstreckt, der mehrere voneinander getrennte Leiterplättchen (P1, P2, P3, P4, P5) umfasst, wobei die Leiterplättchen (P1, P2, P3, P4, P5) über einer gemeinsamen leitenden Platte (D) angeordnet sind, wobei die Leiterplättchen (P1, P2, P3, P4, P5) in Abwesenheit einer Spannungsbelastung durch Isolierstege (C) von der gemeinsamen leitenden Platte (D) beabstandet sind, sodass bei Einwirkung von Druck auf das flexible äußere Substrat (S1) mindestens eines der Leiterplättchen (P1, P2, P3, P4, P5) mit der gemeinsamen leitenden Platte (D) in Kontakt kommt, wodurch mindestens ein Kurzschlusssignal erzeugt wird.

2. Applikator nach Anspruch 1, bei dem mindestens entweder eines der Leiterplättchen (P1, P2, P3, P4, P5) oder einer der Isolierstege (C) elastisch ist.

3. Applikator nach Anspruch 1 oder 2, bei dem die gemeinsame leitende Platte (D) auf einem starren Basissubstrat (S2) angebracht ist.

4. Applikator nach einem der vorhergehenden Ansprüche, bei dem die äußere Kontaktfläche (S11) ein nicht eben ausgeführtes Profil, insbesondere ein gekrümmtes Profil, aufweist.

5. Applikator nach einem der vorhergehenden Ansprüche, der für elektromagnetische Strahlung und Wärme durchlässig ist.

6. Applikator nach einem der vorhergehenden Ansprüche, bei dem der Erfassungsbereich (Z) eine Gesamtfläche in der Größenordnung von 300 mm² bis 400 mm² aufweist, wobei die Leiterplättchen (P1, P2, P3, P4, P5) kleiner als 100 mm² sind.

7. Applikator nach einem der vorhergehenden Ansprüche, bei dem die äußere Kontaktfläche (S11) eine Applikationsfläche für ein flüssiges Produkt darstellt, mit der die Auftragung eines flüssigen Produkts auf eine Zielfläche, beispielsweise die Haut, erfolgt.

8. Applikator nach einem der vorhergehenden Ansprüche, umfassend eine Wand zum Auftragen eines flüssigen Produkts, die in Kontakt mit der äußeren Kontaktfläche angeordnet ist.

9. Applikator nach einem der vorhergehenden Ansprüche, umfassend eine Aktivierungsquelle (51) für das flüssige Produkt und/oder die Haut und eine integrierte oder exportierte Verarbeitungssoftware (L), die geeignet ist, die Kurzschlusssignale zu empfangen und zu verarbeiten, um mindestens eine der folgenden Informationen zu erhalten:
- Identifizierung der kurzgeschlossenen Leiterplättchen (P1, P2, P3, P4, P5),
- Identifizierung der Leiterplättchen (P1, P2, P3, P4, P5), die im Erfassungsbereich (Z) kurzgeschlossen sind,
- Anzahl der Leiterplättchen (P1, P2, P3, P4, P5), die gleichzeitig kurzgeschlossen sind,
- Dauer des Kurzschlusses eines jeden kurzgeschlossenen Plättchens (P1, P2, P3, P4, P5),
- Unterbrechung des Kurzschlusses an jedem kurzgeschlossenen Leiterplättchen (P1, P2, P3, P4, PS),
um mindestens eine der folgenden Aktionen umzusetzen:
- Auslösen der Aktivierungsquelle (51), sobald eine Schwellenzahl von gleichzeitigen Kurzschluss-Signalen erfasst wurde, vorteilhafterweise für eine feste Zeitdauer,
- Abschalten der Aktivierungsquelle (51), sobald eine bestimmte Anzahl von gleichzeitigen Kurzschluss-Signalen nicht mehr erfasst wird,
- Anzeige von Informationen über die Identifizierung, die Position, die Anzahl und/oder die Dauer der Kurzschlüsse auf einem Bildschirm (R), beispielsweise eines Smartphones (Q), beispielsweise in Form einer virtuellen Darstellung (Z') des Erfassungsbereichs (Z) mit seinen Leiterplättchen (P1, P2, P3, P4, PS).

10. Applikator nach Anspruch 9, bei dem die Verarbeitungssoftware (L) derart ausgelegt ist, dass sie bei Empfang einer Schwellenanzahl von Kurzschluss-Signalen ein Signal für größerflächigen Kontakt auslöst, wobei diese Schwellenanzahl mindestens gleich 2 und höchstens gleich der Anzahl der Leiterplättchen (P1, P2, P3, P4, PS) ist.

## Claims

1. A fluid applicator including a contact detector device for detecting contact, and in particular direct or indirect contact with the skin, the applicator being **characterized in that** the contact detector device includes a flexible outer substrate (S1) that defines an outer contact surface (S11), the flexible outer substrate (S1) covering a detection zone (Z) that comprises a plurality of conductive chips (P1, P2, P3, P4, P5) that are separate from one another, the conductive chips (P1, P2, P3, P4, P5) being arranged above a common conductive plate (D), the conductive chips (P1, P2, P3, P4, P5), in the absence of stress, being spaced apart from the common conductive plate (D) by insulating spacers (C), so that pressure exerted on the flexible outer substrate (S1) causes at least one of the conductive chips (P1, P2, P3, P4, P5) to come into contact with the common conductive plate (D), thereby creating at least one short-circuit signal.

2. An applicator according to claim 1, wherein at least one of the conductive chips (P1, P2, P3, P4, P5) and of the insulating spacers (C) is flexible.

3. An applicator according to claim 1 or claim 2, wherein the common conductive plate (D) is mounted on a rigid base substrate (S2).

4. An applicator according to any preceding claim, wherein the outer contact surface (S11) presents a profile other than plane, in particular a curved profile.

5. An applicator according to any preceding claim, being transparent to electromagnetic radiation and to heat.

6. An applicator according to any preceding claim, wherein the detection zone (Z) presents a total surface area lying in the range about 300 mm² to about 400 mm², with conductive chips (P1, P2, P3, P4, P5) of less than 100 mm².

7. An applicator according to any preceding claim, wherein the outer contact surface (S11) forms a fluid applicator surface for applying fluid on a target surface, such as the skin.

8. An applicator according to any preceding claim, including a fluid applicator wall that is arranged in contact with the outer contact surface.

9. An applicator according to any preceding claim, including an activation source (51) for activating the fluid and/or the skin, and integrated or exported processing software (L) that is adapted to receive the short-circuit signals and to processes them so as to obtain at least some of the following information:
· identification of the short-circuited conductive pellets (P1, P2, P3, P4, P5),
· location of the short-circuited conductive chips (P1, P2, P3, P4, P5) in the detection zone (Z),
· number of conductive chips (P1, P2, P3, P4, P5) that are short circuited simultaneously,
· duration of the short circuit of each short-circuited conductive chip (P1, P2, P3, P4, P5),
· breaking of the short circuit of each short-circuited conductive chip (P1, P2, P3, P4, P5),
so as to deduce therefrom at least one of the following actions:
· triggering the activation source (51) whenever a threshold number of simultaneous short-circuit signals is detected, and advantageously for a fixed period of time,
· interrupting the activation source (51) whenever a threshold number of simultaneous short-circuit signals is no longer detected,
· displaying on a screen (R), such as a smartphone (Q), information relating to the identities, the locations, the number, and/or durations of the short circuits, e.g. in the form of a virtual representation (Z') of the detection zone (Z) with its conductive chips (P1, P2, P3, P4, P5).

10. An applicator according to claim 9, wherein the processing software (L) is adapted to deliver an extended-contact signal when it receives some threshold number of short-circuit signals, the threshold number being at least equal to two and at most equal to the number of conductive chips (P1, P2, P3, P4, P5).
